(19) Europäisches Patentamt
European Patent Office
Office européen des brevets

(11) Veröffentlichungsnummer: **0 193 799 B1**

(12) **EUROPÄISCHE PATENTSCHRIFT**

(45) Veröffentlichungstag der Patentschrift: **21.08.91**

(21) Anmeldenummer: **86102201.0**

(22) Anmeldetag: **20.02.86**

(51) Int. Cl.⁵: **C07C 67/10**, C07C 67/03, C07C 67/02, C07C 69/14, C07C 69/54, C07C 69/78, C07C 69/06

# (54) Esteraustauschreaktionen an zeolithischen Katalysatoren.

(30) Priorität: **26.02.85 DE 3506632**

(43) Veröffentlichungstag der Anmeldung:
**10.09.86 Patentblatt 86/37**

(45) Bekanntmachung des Hinweises auf die Patenterteilung:
**21.08.91 Patentblatt 91/34**

(84) Benannte Vertragsstaaten:
**BE CH DE FR GB IT LI NL SE**

(56) Entgegenhaltungen:
**DE-A- 2 510 531**
**US-A- 2 862 962**
**US-A- 3 328 439**

**RECUEIL, Band 89, 1970, Seiten 193-210; D.P. ROELOFSEN et al.: "Ester interchange using molecular sieves"**

(73) Patentinhaber: **BASF Aktiengesellschaft**
**Carl-Bosch-Strasse 38**
**W-6700 Ludwigshafen(DE)**

(72) Erfinder: **Eckhardt, Heinz, Dr.**
**Rüdigerstrasse 7**
**W-6700 Ludwigshafen(DE)**
Erfinder: **Halbritter, Klaus, Dr.**
**Schwarzwaldstrasse 19**
**W-6800 Mannheim 1(DE)**
Erfinder: **Hölderich, Wolfgang, Dr.**
**Mannheimer Strasse 18c**
**W-6710 Frankenthal(DE)**
Erfinder: **Mross, Wolf Dieter, Dr.**
**Anselm-Feuerbach-Strasse 21**
**W-6710 Frankenthal(DE)**

**EP 0 193 799 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Durchführung von Esteraustauschreaktionen, wobei Carbonsäureester mit einer Carbonsäure, einem Alkohol oder einem anderen Carbonsäureester in Gegenwart von aciden zeolithischen Katalysatoren umgesetzt werden.

Unter Esteraustauschreaktionen versteht man Umsetzungen eines Carbonsäureesters mit einer anderen Verbindung, wobei ein vom Ausgangsprodukt verschiedener Ester gebildet wird. Der Reaktionspartner kann eine Carbonsäure, ein Alkohol oder ein weiterer Carbonsäureester sein, dementsprechend wird die Reaktion als Acidolyse, Alkoholyse oder Umesterung bezeichnet wie folgendes Reaktionsschema zeigt:

a) Acidolyse:

$R^1\text{-}COOR^2 + R^3\text{-}COOH = R^3\text{-}COOR^2 + R^1\text{-}COOH$

b) Alkoholyse:

$R^1\text{-}COOR^2 + R^3\text{-}OH = R^1\text{-}COOR^3 + R^2\text{-}OH$

c) Umesterung:

$R^1\text{-}COOR^2 + R^3\text{-}COOR^4 = R^1\text{-}COOR^4 + R^3\text{-}COOR^2$

Eine Zusammenfassung dieser Umsetzungen findet sich in Houben-Weyl, Methoden der organischen Chemie, 4. Aufl., Band 8, S. 516-531. Die Darstellung von Carbonsäureestern auf den obengenannten Wegen ist dann von technischem Interesse, wenn die direkte Veresterung der Carbonsäure mit dem jeweiligen Alkohol nicht möglich oder unwirtschaftlich ist. Gelegentlich kann auch die Bildung des Begleitproduktes (Carbonsäure, Alkohol oder Ester) einen wirtschaftlichen Vorteil bringen, wenn zwei erwünschte Produkte in einer einzigen Reaktion hergestellt werden können.

Eine entscheidende Einschränkung in der Anwendung dieser Esteraustauschreaktionen liegt jedoch darin, daß es sich in jedem Fall um Gleichgewichtsreaktionen handelt, wobei je nach Art der gewählten Reaktionspartner das Ausgangsprodukt oder das Endprodukt im Gleichgewichtszustand dominieren kann. In der Technik führt man solche Reaktionen bevorzugt so durch, daß man eines der Endprodukte ständig - in der Regel durch Destillation - aus dem Reaktionsgemisch entfernt. Die Anwendung ist damit beschränkt auf solche Systeme, in denen ein Endprodukt den niedrigsten Siedepunkt hat. Andernfalls wäre eine Neutralisation des Katalysators und eine (destillative) Trennung des Reaktionsgemisches unter Rückführung der nicht umgesetzten Ausgangsstoffe notwendig. Oft hat aber einer der Ausgangsstoffe den niedrigsten Siedepunkt, so daß eine Verschiebung des Gleichgewichtes durch Abdestillieren eines Endproduktes nicht möglich ist. Das ist z.B. der Fall, wenn niedrig siedende technische Großprodukte verwendet werden, die auf anderem Wege leicht hergestellt werden können, z.B. Methyl- oder Ethylformiat, Methyl- oder Ethylacetat, Methanol oder Ethanol.

Umsetzungen in der Art einer Esteraustauschreaktion sind mit diesen Edukten nur bei Verwendung eines heterogenen Katalysators wirtschaftlich. In US-Patent 3 328 439 werden zu diesem Zweck bestimmte Aluminiumsilikate vorgeschlagen, die durch die folgenden Eigenschaften gekennzeichnet sind: Es sind Zeolithe mit Faujasit-Struktur, die eine Porengröße von 6 bis 13 $\cdot 10^{-10}$ m aufweisen und mit Calzium oder einem Selten-Erdmetall dotiert sind. Die dort angeführten Beispiele gehören jedoch alle zu der Gruppe derjenigen Umsetzungen, bei denen eines der Endprodukte den niedrigsten Siedepunkt besitzt, so daß ihre Eignung bei Problemfällen nicht nachgewiesen ist (siehe Vergleichsbeispiele).

Der Erfindung lag daher die Aufgabe zugrunde, einen heterogenen Katalysator zu finden, der insbesondere die einfache und wirtschaftliche Umsetzung der niedermolekularen Ester und Alkohole ermöglicht. Die Katalysatoren sollten sich durch lange Standzeiten, hohe Aktivität und leichte Regenerierbarkeit auszeichnen und hohe Umsätze bei möglichst niedriger Temperatur mit guter Selektivität gewährleisten.

Es wurde nun gefunden, daß sich Esteraustauschreaktionen, wobei Carbonsäureester mit einer Carbonsäure, einem Alkohol oder einem anderen Carbonsäureester unter Bildung eines Esters mit einer anderen als der ursprünglichen Säure- oder Alkoholkomponente in Gegenwart von Zeolithen umgesetzt werden, und Trennung des entstandenen Gemisches vorteilhaft in der Weise durchführen lassen, daß man die Esteraustauschreaktion in Gegenwart von aciden zeolithischen Katalysatoren des Pentasiltyps ausführt.

Die obengenannten, an die Katalysatoren gestellten Anforderungen werden nach dem erfindungsgemäßen Verfahren weitgehend erfüllt. Besonders vorteilhaft können niedermolekulare Ester, z.B. Methylformiat oder -acetat umgesetzt werden. Auch lassen sich Alkoholysen leicht in Gegenwart eines niedermolekularen Alkohols durchführen. Ausgangsstoffe, die aufgrund der Herstellungsweise noch geringe Mengen an Wasser oder Alkohol enthalten, können ohne weitere Reinigung verwendet werden, wobei diese Nebenkomponenten die Esteraustauschreaktion sogar beschleunigen können.

Zeolithe sind kristalline Aluminosilikate, die eine hochgeordnete Struktur mit einem starren dreidimensionalen Netzwerk von $SiO_4$- und $AlO_4$-Tetraedern besitzen, die durch gemeinsame Sauerstoffatome verbunden sind. Das Verhältnis der Si- und Al-Atome zu Sauerstoff beträgt 1:2. Die Elektrovalenz der

Aluminium enthaltenden Tetraeder ist durch Einschluß von Kationen in den Kristall, z.B. eines Alkali- oder Wasserstoffions ausgeglichen. Ein Kationenaustausch ist möglich. Die Räume zwischen den Tetraedern sind vor der Dehydratation durch Trocknen bzw. Calcinieren von Wassermolekeln besetzt.

Entsprechend ihrer Struktur werden Zeolithe in verschiedene Gruppen unterteilt (siehe Ullmanns Encyclopädie d. techn. Chemie, 4. Aufl., Bd. 24, S. 575-577 (1983)). So bilden bei der Mordenit-Gruppe Ketten oder bei der Chabasit-Gruppe Schichten aus Tetraedern die Zeolith-Struktur, während sich bei der Faujasit-Gruppe die Tetraeder zu Polyedern ordnen, z.B. in Form eines Kuboktaeders, der aus Vierringen bzw. Sechsringen aufgebaut ist. Je nach Verknüpfung der Kuboktaeder, wodurch unterschiedlich große Hohlräume und Poren entstehen, unterscheidet man in Zeolithe vom Typ A, X oder Y.

Nach dem erfindungsgemäßen Verfahren werden Zeolithe dem Pentasiltyps als Katalysatoren verwendet, die als Grundbaustein ein aus $SiO_4$-Tetraedernaufgebauten Fünfring gemeinsam haben. Sie sind durch ein hohes $SiO_2/Al_2O_3$-Verhältnis gekennzeichnet, sowie durch Porengrößen, die zwischen denen der Zeolithe vom Typ A und denen vom Typ X oder Y liegen (vgl. Ullmanns Encyclopädie d. techn. Chem., 4. Aufl., Bd. 24, 1983).

Diese Zeolithe können unterschiedliche chemische Zusammensetzung aufweisen. Es handelt sich hierbei um Alumino-, Boro-, Eisen-, Gallium-, Chrom-, Arsen- und Bismutsilikatzeolithe oder deren Gemische sowie um Alumino-, Boro-, Gallium- und Eisengermanatzeolithe oder deren Gemische.

Insbesondere eignen sich die Alumino-, Boro- und Eisensilikatzeolithe des Pentasiltyps für die Esteraustauschreaktionen. Der Aluminosilikatzeolith wird z.B. aus einer Aluminiumverbindung, vorzugsweise $Al(OH)_3$ oder $Al_2(SO_4)_3$ und einer Siliciumkomponente, vorzugsweise hochdispersem Siliciumdioxid in wäßriger Aminlösung, insbesondere in 1,6-Hexandiamin- oder 1,3-Propandiamin- oder Triethylentetramin-Lösung mit und ohne Alkali-oder Erdalkalizusatz bei 100 bis 220 °C unter autogenem Druck hergestellt. Die erhaltenen Aluminosilikatzeolithe enthalten je nach Wahl der Einsatzstoffmengen ein $SiO_2/Al_2O_3$-Verhältnis von 10 bis 40.000, insbesondere 30 bis 1000. Auch lassen sich derartige Aluminosilikatzeolithe in etherischem Medium wie Diethylenglykoldimethylether, in alkoholischem Medium wie Methanol bzw. 1,4-Butandiol oder in Wasser synthetisieren.

Der Borosilikatzeolith wird z.B. bei 90 bis 200 °C unter autogenem Druck synthetisiert, indem man eine Borverbindung, z.B. $H_3BO_3$, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit und ohne Alkali- oder Erdalkalizusatz zur Reaktion bringt. Solche Borosilikatzeolithe können ebenfalls hergestellt werden, wenn man die Reaktion statt in wäßriger Aminlösung in etherischer Lösung, z.B. Diethylenglykoldimethylether oder in alkoholischer Lösung, z.B. 1,6-Hexandiol durchführt.

Den Eisensilikatzeolith erhält man z.B. aus einer Eisenverbindung, vorzugsweise $Fe_2(SO_4)_3$ und einer Siliciumverbindung, vorzugsweise hochdispersem Siliciumdioxid in wäßriger Aminlösung, insbesondere 1,6-Hexandiamin, mit und ohne Alkali- oder Erdalkalizusatz bei 100 bis 200 °C unter autogenem Druck.

Die so hergestellten Alumino-, Boro- und Eisensilikatzeolithe können nach ihrer Isolierung, Trocknung bei 100 bis 160 °C, vorzugsweise 110 °C, und Calcinierung bei 450 bis 550 °C, vorzugsweise 500 °C, mit einem Bindemittel im Verhältnis 90:10 bis 40:60 Gew.% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25:75 bis 90:5, bevorzugt 75:25, Siliciumdioxid, bevorzugt hochdisperses $SiO_2$, Gemische aus hochdispersem $SiO_2$ und hochdispersem $Al_2O_3$, hochdisperses $TiO_2$ sowie Ton. Nach der Verformung werden die Extrudate oder Preßlinge bei 110 °C/16 h getrocknet und bei 500 °C/16 h calciniert.

Man erhält auch vorteilhafte Katalysatoren, wenn der isolierte Alumino- bzw. Boro- bzw. Eisensilikatzeolith direkt nach der Trocknung verformt wird und erstmals nach der Verformung einer Calcinierung unterworfen wird. Die hergestellten Alumino-, Boro- und Eisensilikatzeolithe können aber auch in reiner Form, ohne Binder, als Stränge oder Tabletten eingesetzt werden.

Liegt der Zeolith aufgrund der Art seiner Herstellung nicht in der katalytisch aktiven, aciden H-Form vor, sondern z.B. in der Na-Form, dann kann diese durch Ionenaustausch mit Ammoniumionen und anschließende Calcinierung oder durch Behandlung mit Säuren vollkommen oder partiell in die gewünschte H-Form überführt werden.

Da bei der erfindungsgemäßen Verwendung der zeolithischen Katalysatoren eine durch Koksabscheidung bedingte Desaktivierung eintreten kann, empfiehlt es sich, die Zeolithe durch Abbrennen der Koksablagerung mit Luft oder mit einem Luft/$N_2$-Gemisch bei 400 bis 550 °C, bevorzugt 500 °C, zu regenerieren. Die Zeolithe erhalten dadurch ihre Anfangsaktivität zurück.

Zur Erhöhung der Selektivität, der Standzeit und der Anzahl an Regenerierungen kann es nützlich sein, Modifizierungen an den zeolithischen Katalysatoren vorzunehmen. Eine Modifizierung der Katalysatoren besteht z.B. darin, daß man den unverformten oder verformten Zeolithen mit Alkalimetallen wie Na - sofern nicht schon von der Synthese her die Alkali-Form des Zeolithen vorliegt - mit Erdalkali wie Ca, Mg und

Erdmetallen wie B, Tl dotiert, z.B. durch einen Ionenaustausch oder durch Imprägnierung mit den entsprechenden Metallsalzen.

Durch eine Dotierung der Zeolithe mit Übergangsmetallen wie W, Fe, Zn, mit Edelmetallen wie Pd und mit seltenen Erdmetallen wie Ce, La kann man besonders vorteilhafte Katalysatoren erhalten. Zweckmäßigerweise führt man die Dotierung z.B. so durch, daß man den verformten Pentasilzeolithen in einem Steigrohr vorlegt und bei 20 bis 100° C z.B. eine wäßrige Lösung eines Halogenids oder eines Nitrats der voranbeschriebenen Metalle überleitet. Ein derartiger Ionenaustausch kann z.B. an der Wasserstoff-, Ammonium- und Alkaliform des Zeolithen vorgenommen werden. Eine weitere Möglichkeit der Metallaufbringung auf den Zeolithen ist gegeben, indem man das zeolithische Material z.B. mit einem Halogenid, einem Nitrat oder einem Oxid der voranbeschriebenen Metalle in wäßriger oder alkoholischer Lösung imprägniert. Sowohl an einem Ionenaustausch als auch an eine Imprägnierung schließt sich zumindest eine Trocknung, wahlweise eine abermalige Calcinierung an.

Eine mögliche Ausführungsform besteht z.B. darin, daß man Wolframsäure $H_2WO_4$ oder $Ce(NO_3)_3 \cdot 6 H_2O$ oder $La(NO_3)_3 \cdot 6 H_2O$ in Wasser - größtenteils zumindest - löst. Mit dieser Lösung wird der verformte oder unverformte Zeolith eine gewisse Zeit, in der Regel ca. 30 min., getränkt. Die eventuell überstehende Lösung wird am Rotationsverdampfer von Wasser befreit. Danach wird der getränkte Zeolith bei ca. 150° C getrocknet und bei ca. 550° C calciniert. Dieser Tränkvorgang kann mehrmals hintereinander vorgenommen werden, um den gewünschten Metallgehalt einzustellen.

Auch ist es möglich, z.B. eine wäßrige $Ce(NO_3)_3$-Lösung herzustellen und darin den reinen pulverförmigen Zeolithen bei 40 bis 100° C unter Rühren ca. 24 h aufzuschlämmen. Nach Abfiltrieren, Trocknen bei ca. 150° C und Calcinierung bei ca. 500° C kann das so gewonnene zeolithische Material mit und ohne Bindemittel zu Strängen, Pellets oder Wirbelgut weiterverarbeitet werden.

Ein Ionenaustausch des in der H-Form oder Ammonium-Form vorliegenden Zeolithen kann z.B. so vorgenommen werden, daß man den Zeolithen in Strängen oder Pellets in einer Kolonne vorlegt und darüber z.B. eine wäßrige $Ce(NO_3)_3$-Lösung bei leicht erhöhter Temperatur zwischen 30 und 80° C im Kreislauf 15 bis 20 h leitet. Danach wird mit Wasser ausgewaschen, bei ca. 150° C getrocknet und bei ca. 550° C calciniert.

Bei manchen metalldotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder mit Wasserdampf unterwirft. Auch kann durch partielle Verkokung (pre-coke) die Aktivität des Katalysators für ein Selektivitätsoptimum des gewünschten Reaktionsproduktes eingestellt werden.

Die hier beschriebenen Katalysatoren können wahlweise als 2 bis 4 mm Stränge oder als Tabletten mit 3 bis 5 mm Durchmesser oder als Pulver mit Teilchengrößen von 0,1 bis 0,5 mm oder als Wirbelkontakt eingesetzt werden.

Obwohl die erfindungsgemäßen Katalysatoren ihre besonderen Vorteile bei der Umsetzung der niedermolekularen Ester oder Alkohole haben, können sie natürlich auch vorteilhaft dann eingesetzt werden, wenn ein Reaktionsprodukt den niedrigsten Siedepunkt hat und das Gleichgewicht leicht zu vollständigem Umsatz hin verschoben werden kann. Im Vergleich mit den Faujasit-Zeolithen ergeben die erfindungsgemäßen Pentasil-Zeolithe bei niedrigeren Temperaturen höhere Umsätze mit sehr hoher Selektivität für die Esteraustauschreaktionen.

Einfache Beispiele für die drei genannten Reaktionstypen sind Umsetzungen nach den folgenden Reaktionsgleichungen:

$$1)\quad H\text{-}COOCH_3 + CH_3COOH \rightleftharpoons CH_3COOCH_3 + HCOOH$$

$$2)\quad H\text{-}COOCH_3 + t\text{-}Bu\text{-}OH \rightleftharpoons H\text{-}COO\text{-}t\text{-}Bu + CH_3OH$$

$$3)\quad H\text{-}COOCH_3 + CH_3\text{-}COOC_2H_5 \rightleftharpoons H\text{-}COOC_2H_5 + CH_3\text{-}COOCH_3$$

An die chemische Reinheit der Ausgangsstoffe sind keine besonderen Anforderungen zu stellen. Technisch hergestellte Carbonsäuren und Ester können noch geringe Mengen an Alkohol oder Wasser enthalten. Diese Nebenkomponenten können die Esteraustauschreaktionen bis zu einem gewissen Grade beschleunigen. Aus diesem Grunde ist es oft wünschenswert, daß im Reaktionsgemisch 0.1 bis 5,0 Gew.% Wasser oder ein Alkohol der Formel $R^2$-OH oder $R^4$-OH, worin $R^2$ und $R^4$ der im Umsetzungsgemisch vorhandenen Alkoholkomponente des Esters (siehe Reaktionsgleichungen 1) und 3)) entspricht, enthalten ist.

Im allgemeinen können die Reste $R^1$ bis $R^4$ des auf Seite 1 angegebenen Reaktionsschemas sehr breit

variiert werden. Sie stehen z.B. für aliphatische, cycloaliphatische, araliphatische oder aromatische Reste und insbesondere für Wasserstoff, Alkyl-, Alkenyl-, Alkinyl-, Cycloalkyl oder Arylgruppen. Die Kohlenstoffzahl der organischen Reste liegt in der Regel bei 1 bis 30. Besonders vorteilhaft läßt sich das erfindungsgemäße Verfahren anwenden, wenn Ester umgesetzt werden sollen, bei denen $R^2$ eine Methyl-oder Ethylgruppe darstellt und $R^1$ für Wasserstoff oder eine Methyl- oder Ethylgruppe steht.

Als Reaktionspartner kommen für die Alkoholyse sowohl hoch- als auch niedermolekulare Alkohole in Betracht, die gesättigt oder ungesättigt, ein- oder mehrwertig sein können, z.B. Dodekanol-(1), Hexadekanol-(1), Allylalkohol, Phenol, Benzylalkohol, Ethylenglykol oder Glycerin. Besonders geeignet sind Alkohole mit 1 bis 15, insbesondere 1 bis 10, vorzugsweise 1 bis 5 Kohlenstoffatomen z.B. Methanol, Ethanol, n-Propanol, i-Propanol, n-Butanol, i-Butanol, s-Butanol, t-Butanol, n-Pentanol, n-Hexanol, Cyclohexanol oder 2-Ethylhexanol.

Für die Acidolyse geeignete Säuren sind aliphatische oder aromatische Mono- oder Polycarbonsäuren, z.B. Crotonsäure, Pivalinsäure, Laurinsäure, Palmitinsäure, Citronensäure, Salicylsäure, Fumarsäure, Malonsäure, Glutarsäure, Phthal- oder Terephthalsäure. Besonders bevorzugt sind aliphatische Carbonsäuren mit 1 bis 10, insbesondere 1 bis 5 Kohlenstoffatomen, z.B. Ameisensäure, Essigsäure, Propionsäure, Buttersäure, Acryl- oder Methacrylsäure oder aromatische Carbonsäuren wie Benzoesäure.

Für die Umesterung c) in Betracht kommende Ester sind solche der o.g. Säuren. Besonders vorteilhaft läßt sich das erfindungsgemäße Verfahren auf niedermolekulare Ester wie Methyl- und Ethylformiat oder Methyl- und Ethylacetat anwenden.

Es ist möglich, die Umsetzungen diskontinuierlich durchzuführen, indem z.B. pulverförmiger Katalysator in einem Rührbehälter suspendiert und das Reaktionsgemisch 2 bis 20 h bei Reaktionstemperatur gerührt wird. Die optimale Reaktionstemperatur wird durch die Wahl der Ausgangsstoffe gegeben und liegt in der Regel oberhalb von 0 °C, meist zwischen 30 °C und 400 °C, insbesondere zwischen 30 und 250 °C.

Die Reaktion wird in der Regel bei Atmosphärendruck oder Eigendruck der Reaktionsteilnehmer durchgeführt; man kann auch zusätzlich ein Inertgas verwenden, wenn ein bestimmter Druck gewünscht ist.

Eine bevorzugte Art der Reaktionsführung ist die kontinuierliche. Dabei kann man so verfahren, daß man ein Reaktionsrohr mit verformtem Katalysator füllt und die umzusetzende Mischung mit einer mittleren Verweilzeit von 5 bis 100 min bei der gewünschten Reaktionstemperatur von 0 bis 400, insbesondere 30 bis 400, vorzugsweise 30 bis 250 °C, flüssig durch das Rohr pumpt, wobei man das umzusetzende Gemisch mit einer mittleren Verweilzeit von 1 bis 300 sec gasförmig durch einen mit verformtem Katalysator gefüllten Rohrrekator leitet, Die Reaktionsgemische, die im optimalen Fall einem Gleichgewichtszustand entsprechen, können dann nach üblichen Methoden, z.B. destillativ, getrennt werden. Die nichtumgesetzten Ausgangsstoffe können - falls gewünscht - wieder zurückgeführt werden.

Die Esteraustauschreaktionen lassen sich aber auch in der Gasphase ausführen. Die hierfür gewählten Reaktionsbedingungen sind 100 bis 300 °C, vorzugsweise 130 bis 250 °C, und eine Belastung WHSV = 0,1 bis 20 h-1, bevorzugt 0,5 bis 5 $h^{-1}$ (g Einsatzgemisch/g Katalysator und Stunde). Das Verfahren kann chargenweise oder kontinuierlich, drucklos oder unter Druck durchgeführt werden. Nichtumgesetzte Einsatzkomponenten können gegebenenfalls nach der Umsetzung in an sich bekannter Weise von den gewünschten Produkten abgetrennt und erneut für die erfindungsgemäße Umsetzung verwendet werden.

Die folgenden Beispiele veranschaulichen die Erfindung. Die benutzte Abkürzung GC steht für Gaschromatographie bzw. Gaschromatogramm.

Die für die erfindungsgemäßen Esteraustauschreaktionen eingesetzten Katalysatoren sind:

Katalysator A

Ein Aluminosilikatzeolith vom Pentasil-Typ wurde unter hydrothermalen Bedingungen bei autogenem Druck und 150 °C aus 650 g hochdispersem $SiO_2$, 203 g $Al_2(SO_4)_3 \cdot 18 H_2O$ in 10 kg einer wäßrigen 1.6-Hexandiamin-Lösung (Mischung 50:50 Gew.%) in einem Rührautoklaven hergestellt. Nach Abfiltrieren und Auswaschen wurde das kristalline Reaktionsprodukt bei 110 °C/24 h getrocknet und bei 500 °C/24 h calcinlert. Dieser Aluminosilikatzeolith enthielt 92,8 Gew.% $SiO_2$ und 4,2 Gew.% $Al_2O_3$.

Katalysator B

Katalysator B wurde erhalten durch Verformung des Aluminosilikatzeolithen (Katalysator A) mit Boehmit (Gewichtsverhältnis 60:40) zu 2 mm-Strängen, die bei 110 °C/16 h getrocknet und bei 500 °C/16 h calciniert wurden.

Katalysator C

Durch Imprägnieren des Katalysators B mit einer wäßrigen $La(NO_3)_3$-Lösung und anschließender Trocknung (130 °C/2 h) bzw. Calcination (540 °C/2 h) wurde Katalysator C hergestellt. Der La-Gehalt betrug 3,5 Gew.%.

Katalysator D

Katalysator B wurde mit einer wäßrigen Wolframoxid-Lösung dotiert, bei 130 °C/2 h getrocknet und bei 540 °C/2 h calciniert. Dieser Katalysator D enthielt 4,0 Gew.% W.

Katalysator E

Katalysator E wurde hergestellt, indem man eine wäßrige $Ce(NO_3)_3$-Lösung auf Katalysator B aufbrachte, bei 130 °C/2 h trocknete und bei 540 °C/2 h calcinierte. Katalysator C besaß 6,2 Gew.% Ce.

Katalysator F

75 g des Aluminosilikatzeolithen (Katalysator A) wurden mit 210 ml einer 0,1 n HF in 60 ml $H_2O$ 1 h unter Rückfluß behandelt. Das abfiltrierte Produkt wurde gewaschen, bei 110 °C/16 h getrocknet und bei 500 °C/5 h calciniert. Dieses Pulver wurde mit amorphem Aluminosilikat ($SiO_2$:$Al_2O_3$ = 75:25 Gew.%) im Gewichtsverhältnis 60:40 zu 2,5 mm-Strängen verformt, bei 110 °C/16 h getrocknet und bei 500 °C/16 h calciniert.

Katalysator G

Der Aluminosilikatzeolith des Katalysators G wurde wie Katalysator A hergestellt, jedoch wurden 80 g hochdisperses $SiO_2$, 5 g $Al(NO_3)_3 \cdot 9\ H_2O$ und 1 g NaOH in 800 g 50 %iger Hexamethylendiamin-Lösung verwendet. Dieser Aluminosilikatzeolith enthielt nach der Trocknung bei 110 °C/16 h und Calcinierung bei 500 °C/16 h 97,5 % $SiO_2$, 0,22 Gew.-% $Al_2O_3$ und 0,13 Gew.% Na. Dieses Pulver wurde mit Boehmit im Gewichtsverhältnis 60:40 zu 2 mm-Strängen verformt, bei 110 °C/16 h getrocknet und bei 500 °C/16 h calciniert.

Die Stränge wurden einem Ionenaustausch mit 20 %iger $NH_4Cl$-Lösung bei 80 °C innerhalb von 2 h unterworfen. Der Austausch wurde mehrmals wiederholt, wobei zwischen den einzelnen Austauschprozessen bei 500 °C calciniert wurde. Hierdurch wurde der Na-Gehalt des Katalysators auf 0,008 Gew.% gesenkt.

Katalysator H

Katalysator H wurde wie Katalysator A hergestellt, jedoch in 50 %iger 1,3-Diaminopropan-Lösung. Dieser Aluminosilikatzeolith enthielt 91,6 Gew.% $SiO_2$ und 3,2 Gew.% $Al_2O_3$.

Katalysator I

Der Eisensilikatzeolith des Pentasil-Typs wurde unter hydrothermalen Bedingungen bei autogenem Druck und 165 °C aus 273 g Wasserglas, gelöst in 253 g einer wäßrigen 1,6-Hexandiamin-Lösung (Mischung 50:50 Gew.%) und 31 g Eisensulfat, gelöst in 21 g 96 %iger Sohwefelsäure und 425 g Wasser, in einen Rührautoklaven während 4 Tagen synthetisiert, danach abfiltriert, ausgewaschen, bei 100 °C/24 h calciniert. Dieser Eisensilikatzeolith zeigte ein $SiO_2/Fe_2O_3$-Verhältnis von 17,7 und einen $Na_2O$-Gehalt von 0,62 Gew.%.

Dieses Pulver wurde zu 4 mm-Strängen verformt, bei 110 °C/16 h getrocknet und bei 500 °C/16 h calciniert. Diese Stränge wurden bei 80 °C/2 h mit einer 20 %igen $NH_4Cl$-Lösung (Gewichtsverhältnis 1:15) behandelt, danach ausgewaschen, bei 110 °C/16 h getrocknet und bei 500 °C/5 h calciniert.

Vergleichskatalysatoren J bis N nach US 3 328 439

Katalysator J ist ein Aluminosilikatzeolith 13 X.

Katalysator K ist ein Aluminosilikatzeolith des Y-Typs.

Katalysator L wurde durch Ionenaustausch des Katalysators J mit wäßriger $Ca(NO_3)_2$-Lösung während 2 h bei 80 °C erhalten.

Katalysator M wurde durch Ionenaustausch des Katalysators J mit wäßriger $La(NO_3)_3$-Lösung während

4 h bei 80 °C erhalten.

Katalysator N wurde durch Ionenaustausch des Katalysators K mit wäßriger $Ca(NO_3)_2$-Lösung während 2 h bei 80 °C erhalten.

Vergleichsbeispiele 1 bis 8

In einem Rührautoklaven wurden 60 g Essigsäure, 60 g techn. Methylformiat und die angegebene Menge Katalysator 5 h bei der angegebenen Temperatur gehalten. Der Reaktionsaustrag wurde gaschromatographisch (GC-Säule: 3 m SE 30; 80 °C; Flammenionisationsdetektor) untersucht, wobei die gebildete Ameisensäure nicht erfaßt wurde. (Die Beispiele 1 bis 3 wurden ohne Katalysator durchgeführt.)

Tabelle 1

| | | | | Produktgemisch in GC-Flächen-% | | |
|---|---|---|---|---|---|---|
| Nr. | Kat. | Menge | Temp. | Methylformiat | Methylacetat | Essigsäure |
| 1 | - | - | 70 | 42,4 | 1,3 | 56,3 |
| 2 | - | - | 90 | 40,8 | 3,0 | 56,2 |
| 3 | - | - | 110 | 35,3 | 11,5 | 53,2 |
| 4 | J | 5 g | 130 | 34,7 | 15,4 | 49,5 |
| 5 | K | 5 g | 130 | 31,5 | 13,0 | 55,5 |
| 6 | L | 5 g | 90 | 40,3 | 4,7 | 55,0 |
| 7 | M | 5 g | 130 | 33,9 | 9,9 | 56,1 |
| 8 | N | 5 g | 90 | 41,7 | 2,0 | 55,5 |

Beispiele 9 bis 23

Es wurde wie in den Beispielen 1 bis 8 verfahren, jedoch die in Tabelle 2 angegebenen Katalysatoren verwendet.

Tabelle 2

| | | | | Produktgemisch in GC-Flächen-% | | |
|---|---|---|---|---|---|---|
| Nr. | Kat. | Menge | Temp. | Methylformiat | Methylacetat | Essigsäure |
| 9 | A | 5 g | 70 | 36,9 | 20,3 | 42,4 |
| 10 | A | 5 g | 110 | 26,8 | 41,0 | 31,2 |
| 11 | A | 5 g | 150 | 21,0 | 49,2 | 28,0 |
| 12 | B | 5 g | 70 | 38,5 | 15,7 | 45,7 |
| 13 | B | 5 g | 130 | 26,3 | 39,4 | 34,0 |
| 14 | C | 5 g | 90 | 35,7 | 26,0 | 38,3 |
| 15 | D | 5 g | 90 | 35,8 | 24,7 | 39,0 |
| 16 | E | 5 g | 90 | 34,3 | 28,0 | 37,4 |
| 17 | E | 5 g | 70 | 38,1 | 16,1 | 45,9 |
| 18 | F | 5 g | 90 | 34,4 | 27,8 | 37,3 |

Tabelle 2: Fortsetzung

| Nr. | Kat. | Menge | Temp. | Produktgemisch in GC-Flächen-% Methylformiat | Methylacetat | Essigsäure |
|---|---|---|---|---|---|---|
| 19 | F | 5 g | 70 | 37,4 | 16,3 | 46,3 |
| 20 | G | 5 g | 90 | 37,3 | 17,9 | 45,5 |
| 21 | H | 5 g | 90 | 34,7 | 23,6 | 41,6 |
| 22 | H | 5 g | 150 | 19,2 | 48,4 | 31,8 |
| 23 | I | 5 g | 90 | 39,0 | 15,8 | 45,1 |

Beispiele 24 bis 27

In einem Rührautoklaven wurden 60 g Essigsäure, 120 g Methylformiat (Methanolgehalt < 0,1 %), 5 g Katalysator B und die in der Tabelle 3 angegebene Menge Methanol 5 h bei 100 °C gehalten. Man erhielt folgende Ergebnisse:

Tabelle 3

| Nr. | g Methanol | Produktgemisch in GC-Flächen-% Methylformiat | Methylacetat | Essigsäure |
|---|---|---|---|---|
| 24 | 0 | 54,2 | 13,3 | 32,2 |
| 25 | 2 | 54,5 | 15,8 | 29,5 |
| 26 | 4 | 49,9 | 21,9 | 26,9 |
| 27 | 6 | 50,8 | 23,0 | 24,8 |

Beispiele 28 bis 34

In einem Rührautoklaven wurden die in der Tabelle 4 angegebenen Ausgangsstoffe X = Methylformiat und Y 5 h bei der angegebenen Temperatur gehalten. Man erhielt folgende Ergebnisse:

Tabelle 4

| Nr. | Kat. | Ausgangsstoffe X | Y | T/°C | Produktgemisch in GC-Flächen-% X | Y | Reaktionsprodukt |
|---|---|---|---|---|---|---|---|
| 28 | 5,3 g A | 60 g | 72 g Acrylsäure | 120 | 18,5 | 39,2 | 31,2; Methylacrylat |
| 29 | 4,8 g A | 60 g | 61 g Benzoesäure | 150 | 29,7 | 34,9 | 31,0; Methylbenzoat |
| 30 | 7,8 g A | 120 g | 74 g n-Butanol | 110 | 17,8 | 46,1 | 27,7; n-Butylformiat<br>6,8; Methanol |

Tabelle 4: Fortsetzung

| Nr. | Kat. | Ausgangsstoffe X | Ausgangsstoffe Y | T/°C | Produktgemisch in GC-Flächen-% X | Y | Reaktionsprodukt |
|---|---|---|---|---|---|---|---|
| 31 | 7,8 g B | 120 g | 74 g t-Butanol | 110 | 24,7 | 37,6 | 16,1; t-Butylformiat<br>18,2; Methanol |
| 32 | 8,4 g A | 120 g | 88 g Ethylacetat | 110 | 26,5 | 28,2 | 23,9; Ethylformiat<br>20,4; Methylacetat |
| Vergleichsbeispiel | | | | | | | |
| 33 | - | 60 g | 61 g Benzoesäure | 110 | | | kein Umsatz |
| 34 | - | 120 g | 74 g t-Butanol | 110 | 23,2 | 76,6 | kein Umsatz |

Beispiel 35

Ein 2,3 l Rohrreaktor wurde mit Katalysator B gefüllt. Bei 130° C wurden stündlich 776 9 Methylformiat, 24 g Methanol und 200 g Essigsäure zudosiert. Der Austrag (998 g/h) hatte folgende Zusammensetzung:

64,2 % Methylformiat
20,0 % Methylacetat
10, 1 % Ameisensäure
3,6 % Essigsäure
0,9 % Wasser
0,6 % Methanol

Das entspricht einem Umsatz an Essigsäure von 81 % bei praktisch quantitativer Selektivität zu Methylacetat.

Beispiel 36

In den gleichen Reaktor wurden stündlich 2.328 g Methylformiat, 72 g Methanol und 600 g Essigsäure zudosiert. Der Austrag (2.996 g/h) hatte folgende Zusammensetzung:

70,8 % Methylformiat
11,8 % Methylacetat
10,2 % Essigsäure
5,1 % Ameisensäure
0,9 % Wasser
0,6 % Methanol

Das entspricht einem Umsatz an Essigsäure von 48 % bei praktisch quantitativer Selektivität zu Methylacetat.

Beispiele 37 bis 40

Die Reaktionen in der Gasphase wurden unter isothermen Bedingungen in einem Rohrreaktor (Wendel, 0,6 cm Innendurchmesser, 90 cm Länge) ca. 8 Stunden lang durchgeführt. Die Reaktionsprodukte wurden destillativ abgetrennt und durch Siedepunkt, Brechungsindex und NMR-Spektren charakterisiert. Die quantitative Bestimmung der Reaktionsprodukte und der Ausgangsprodukte erfolgte gaschromatographisch.

Die bei der Umesterung von Ameisensäuremethylester mit Essigsäure erzielten Ergebnisse sind in Tabelle 5 zusammengestellt. Es wurde technischer Ameisensäuremethylester eingesetzt, der noch 3 bis 5 % Methanol enthält.

Tabelle 5

Umesterung von Ameisensäuremethylester mit Essigsäure (1:1 molar) zu Essigsäuremethylester und Ameisensäure

| | | | | |
|---|---|---|---|---|
| Beispiel | 37 | 38 | 39 | 40 |
| Katalysator | A | A | A | H |
| Temperatur | 130°C | 150°C | 130°C | 110°C |
| WHSV | 1,5 $h^{-1}$ | 1,5 $h^{-1}$ | 6 $h^{-1}$ | 3 $h^{-1}$ |
| Produktgemisch in Flächen-% | | | | |
| Essigsäuremethylester | 22,6 | 30,4 | 23,0 | 29,3 |
| Ameisensäure | 15,1 | 16,0 | 13,7 | 13,1 |
| Ameisensäuremethylester | 14,7 | 13,5 | 25,0 | 18,3 |
| Essigsäure | 41,8 | 32,7 | 34,5 | 34,3 |
| Methanol | 0,5 | 0,6 | 0,5 | 0,5 |
| $H_2O$ | 5,3 | 6,8 | 3,3 | 4,4 |

## Patentansprüche

1. Verfahren zur Durchführung von Esteraustauschreaktionen, wobei Carbonsäureester mit einer Carbonsäure, einem Alkohol oder einem anderen Carbonsäureester unter Bildung eines Esters mit einer anderen als der ursprünglichen Säure- oder Alkoholkomponente in Gegenwart von Zeolithen umgesetzt werden und Trennung des entstandenen Gemisches, dadurch gekennzeichnet, daß man die Esteraustauschreaktion in Gegenwart von aciden zeolithischen Katalysatoren des Pentasiltyps durchführt.

2. Verfahren nach Anspruch 1, dadurch gekennzeichnet, daß man als Katalysatoren Aluminium-, Boro- oder Eisensilikatzeolithe verwendet.

3. Verfahren nach den Ansprüchen 1 und 2, dadurch gekennzeichnet, daß man mit Übergangsmetallen dotierte Zeolithe als Katalysatoren verwendet.

4. Verfahren nach den Ansprüchen 1 und 2, dadurch gekennzeichnet, daß man mit Edelmetallen, seltenen Erdmetallen oder mit Wolfram dotierte Zeolithe als Katalysatoren verwendet.

5. Verfahren nach den Ansprüchen 1 bis 4, dadurch gekennzeichnet, daß man die zeolithischen Katalysatoren mit oder ohne Bindemittel in Form von Strängen, Tabletten, Pulver oder als Wirbelkontakt verwendet.

6. Verfahren nach den Ansprüchen 1 bis 5, dadurch gekennzeichnet, daß man für die Esteraustauschreaktion Ausgangsstoffe mit einem niedrigeren Siedepunkt verwendet als die entstehenden Produkte.

7. Verfahren nach den Ansprüchen 1 bis 6, dadurch gekennzeichnet, daß man die Umsetzung diskontinuierlich durchführt, indem man das umzusetzende Reaktionsgemisch zusammen mit dem darin suspendierten Katalysator bei Temperaturen oberhalb von 0 bis 400°C rührt und das entstandene Reaktionsgemisch nach den dafür üblichen Methoden trennt.

8. Verfahren nach den Ansprüchen 1 bis 6, dadurch gekennzeichnet, daß man die Umsetzung kontinuierlich bei Temperaturen oberhalb von 0 bis 400°C durchführt, das entstandene Reaktionsgemisch nach den dafür üblichen Methoden trennt und gegebenenfalls nicht umgesetzte Ausgangsstoffe wieder der Esteraustauschreaktion zuführt.

9. Verfahren nach Anspruch 8, dadurch gekennzeichnet, daß man das umzusetzende Gemisch mit einer mittleren Verweilzeit von 5 bis 100 min flüssig durch ein mit verformtem Katalysator gefülltes Reaktionsrohr pumpt.

10. Verfahren nach Anspruch 8, dadurch gekennzeichnet, daß man das umzusetzende Gemisch mit einer mittleren Verweilzeit von 1 bis 300 sec gasförmig durch einen mit verformtem Katalysator gefüllten Rohrreaktor leitet.

## Claims

1. A process for carrying out ester exchange reactions, in which a carboxylate is reacted with a carboxylic acid, with an alcohol, or with another carboxylate, with formation of an ester which contains an acid or alcohol component other than the original one, in the presence of a zeolite, and separating the resulting mixture, wherein the ester exchange reaction is carried out in the presence of an acid zeolite catalyst of the Pentasil type.

2. A process as claimed in claim 1, wherein the catalyst used is an aluminosilicate, borosilicate or iron silicate zeolite.

3. A process as claimed in claim 1 or 2, wherein the catalyst used is a zeolite doped with a transition metal.

4. A process as claimed in claim 1 or 2, wherein the catalyst used is a zeolite doped with a noble metal, with a rare earth metal or with tungsten.

5. A process as claimed in any of claims 1 to 4, wherein the zeolite catalyst is used with or without a binder, in the form of extrudates, tablets or a powder or as a fluidized-bed catalyst.

6. A process as claimed in any of claims 1 to 5, wherein starting materials having a lower boiling point than the resulting products are used for the ester exchange reaction.

7. A process as claimed in any of claims 1 to 6, wherein the reaction is carried out batchwise by stirring the reaction mixture to be converted, together with the catalyst suspended therein, at from above 0 to 400 °C, and separating the resulting reaction mixture by a conventional method.

8. A process as claimed in any of claims 1 to 6, wherein the reaction is carried out continuously at from above 0 to 400 °C, the resulting reaction mixture is separated by a conventional method and, if required, unconverted starting materials are recycled to the ester exchange reaction.

9. A process as claimed in claim 8, wherein the mixture to be converted is pumped in liquid form through a reaction tube filled with the molded catalyst, the mean residence time being from 5 to 100 min.

10. A process as claimed in claim 8, wherein the mixture to be converted is passed in gaseous form through a tube reactor filled with the molded catalyst, the mean residence time being from 1 to 300 sec.

## Revendications

1. Procédé pour l'execution de réactions de transestérification dans lesquelles des esters d'acides carboxyliques sont mis en réaction, en presence de zéolithes, avec un acide carboxylique, un alcool ou un autre ester d'acide carboxylique pour former un ester dont le composant acide ou alcool est différent de celui de l'ester initial, puis le mélange forme est séparé, caractérisé en ce qu'on conduit la réaction de transestérification en présence de catalyseurs zéolithiques acides du type pentasil.

2. Procédé selon la revendication 1, caractérisé en ce qu'on utilise, comme catalyseurs, des zéolithes d'alumino-, boro- ou ferrosilicate.

3. Procédé selon la revendication 1 ou 2, caractérisé en ce qu'on utilise, comme catalyseurs, des zéolithes dopées avec des métaux de transition.

4. Procédé selon la revendication 1 ou 2, caractérisé en ce qu'on utilise, comme catalyseurs, des zéolithes dopées avec des métaux nobles, avec des métaux des terres rares ou avec du tunstène.

5. Procédé selon l'une quelconque des revendications 1 à 4, caractérisé en ce qu'on utilise les catalyseurs zéolithiques avec ou sans liant sous forme de bâtonnets, de pastilles, de poudre ou sous forme de catalyseur en lit fluidise.

6. Procédé selon l'une quelconque des revendications 1 à 5, caractérisé en ce qu'on utilise, pour la réaction de transestérification, des substances de départ dont le point d'ébullition est inférieur à celui des produits formés.

7. Procédé selon l'une quelconque des revendications 1 à 6, caractérisé en ce qu'on conduit la réaction de façon discontinue en agitant le mélange réactionnel à faire réagir avec le catalyseur qui y est contenu en suspension, à des températures de 0 à 400 °C, et en séparant le mélange réactionnel formé par des méthodes usuelles à cet effet.

8. Procédé selon l'une quelconque des revendications 1 à 6, caractérisé en ce qu'on conduit la réaction en continu à des températures de 0 à 400 °C, on sépare le mélange réactionnel formé par des méthodes usuelles à cet effet et on recycle éventuellement dans la réaction de transestérification les substances de départ n'ayant pas réagi.

9. Procédé selon la revendication 8, caractérisé en ce qu'on pompe à l'état liquide le mélange à faire reagir à travers un tube de réaction garni de catalyseur façonne, avec un tempe de séjour moyen de 5 à 100 mn.

10. Procédé selon la revendication 8, caractérisé en ce qu'on envoie à l'état gazeux le mélange à faire réagir a travers un tube de réaction garni de catalyseur façonne, avec un tempe de séjour moyen de 1 à 300 s.